# EUROPÄISCHE PATENTSCHRIFT

(11) **EP 4 358 894 B1**
(45) Veröffentlichungstag und Bekanntmachung des Hinweises auf die Patenterteilung: **14.05.2025**
(21) Anmeldenummer: 22741154.3
(22) Anmeldetag: 10.06.2022
(51) Int. Cl.: A61B 90/70

(54) **ADAPTER ZUR KOPPLUNG EINES HOHLKÖRPERS IM MEDIZINISCHEN BEREICH MIT EINEM VORRATSBEHÄLTER FÜR EIN BEHANDLUNGSMITTEL**
ADAPTER FOR COUPLING A HOLLOW BODY IN THE MEDICAL FIELD, COMPRISING A STORAGE CONTAINER FOR A TREATMENT AGENT
ADAPTATEUR POUR ACCOUPLER UN CORPS CREUX DANS LE DOMAINE MÉDICAL AVEC UN RÉSERVOIR DE STOCKAGE POUR UN AGENT DE TRAITEMENT

(30) Priorität: 26.06.2021 DE 102021116565
(43) Veröffentlichungstag der Anmeldung: 01.05.2024
(73) Patentinhaber: IC MEDICAL GMBH, 73635 Rudersberg-Steinberg (DE)
(72) Erfinder: BLUMENSCHEIN, Thomas, 73525 Schwäbisch-Gmünd (DE)
(74) Vertreter: Heidinger, Andreas
(86) Internationale Anmeldenummer: PCT/EP2022/065874
(87) Internationale Veröffentlichungsnummer: WO 2022/268532

(56) Entgegenhaltungen:
- WO-A1-97/48424
- US-A1- 2013 206 869
- US-B1- 6 623 445

## Beschreibung

Die Erfindung betrifft einen Adapter zur Kopplung eines Hohlkörpers im medizinischen Bereich mit einem Vorratsbehälter für ein Behandlungsmittel mit den Merkmalen des Oberbegriffs des Anspruchs 1.

Mehrmals verwendbare Geräte im medizinischen Bereich müssen nach jedem Gebrauch mit einem Reinigungsmittel gereinigt und eventuell zusätzlich mit einem Desinfektionsmittel desinfiziert werden. Ein derartiges Gerät kann über bewegliche Teile verfügen, die regelmäßig mit einem Pflegemittel beispielsweise in Form eines speziellen Pflege-Öls geschmiert werden müssen. Derartige Reinigungs-, Desinfektions- und Pflegemittel können neben weiteren vergleichbaren Mitteln als Behandlungsmittel bezeichnet werden. Die Geräte weisen häufig Kanäle, beispielsweise zum Zuführen- und Weiterleiten von Druckluft auf, weshalb derartige Geräte mit Kanälen als Hohlkörper im medizinischen Bereich bezeichnet werden können.

Typische Beispiele derartiger Hohlkörper im medizinischen Bereich sind so genannte Hand- und Winkelstücke im zahnärztlichen Bereich, welche von Hand geführt und mit einem Elektromotor angetrieben werden. Hohlkörper im medizinischen Bereich können daneben beispielsweise auch als so genannte Turbinen im zahnärztlichen Bereich, ausgeführt sein, welche mit Druckluft angetrieben werden.

Das Behandlungsmittel wird dem Hohlkörper üblicherweise über seinen Druckluftanschluss oder seine so genannte Motorkupplung zugeführt. Der Hohlkörper kann auch andere Öffnungen aufweisen, über die Behandlungsmittel zugeführt werden kann. Das Behandlungsmittel wird dabei meist aus einem unter Druck stehenden Vorratsbehälter in Form einer handelsüblichen, mit einem Gemisch aus Treibgas und Behandlungsmittel gefüllten Spraydose entnommen. Das genannte Gemisch wird im Folgenden vereinfachend als Behandlungsmittel bezeichnet.

Der Vorratsbehälter weist insbesondere eine hauptsächlich zylinderförmige Form mit einer Längsachse in Axialrichtung auf. Der Vorratsbehälter verfügt über ein Ventil, das im geöffneten Zustand Behandlungsmittel aus dem Vorratsbehälter austreten lässt und im geschlossenen Zustand den Vorratsbehälter so verschließt, dass kein Behandlungsmittel austreten kann. Das Ventil verfügt üblicherweise über ein aus dem Vorratsbehälter herausragendes Röhrchen. Durch zumindest teilweises Eindrücken des Röhrchens entlang der Längsachse in Richtung Vorratsbehälter wird das Ventil geöffnet und Behandlungsmittel strömt über das Röhrchen und damit über das Ventil aus dem Vorratsbehälter. Sobald das Röhrchen nicht mehr aktiv in Richtung Vorratsbehälter gedrückt wird, wird es durch eine Druckfeder vom Vorratsbehälter weg gegen einen Ventilsitz des Ventils gedrückt. Damit wird das Ventil wieder geschlossen und Behandlungsmittel kann nicht mehr über das Röhrchen aus dem Vorratsbehälter austreten. Das Öffnen des Ventils kann als eine Betätigung des Ventils des Vorratsbehälters bezeichnet werden. Der Vorratsbehälter kann auch eine andere Form, beispielsweise eine hauptsächlich quaderförmige Grundform aufweisen, wobei die Richtung, in der das Ventil in den Vorratsbehälter zum Öffnen eingedrückt wird, immer als Längsrichtung des Vorratsbehälters angesehen wird.

Um dem Hohlkörper Behandlungsmittel aus dem Vorratsbehälter zuführen zu können, muss der Hohlkörper mit dem Vorratsbehälter gekoppelt und anschließend zusätzlich das Ventil des Vorratsbehälters betätigt werden.

Die EP 2 628 693 B1 beschreibt einen Adapter zur Kopplung eines Hohlkörpers im medizinischen Bereich mit einem Vorratsbehälter für ein Behandlungsmittel in Form einer handelsüblichen Spraydose mit Pflege-Öl. Der Adapter verfügt über ein Halteelement, welches am Vorratsbehälter unbeweglich angeordnet werden kann. Ein hohlförmiges Betätigungselement in Form eines Aktuators ist im Haltelement verschiebbar angeordnet. Das Betätigungselement kann von einem Führungselement geführt in Richtung Vorratsbehälter translatorisch entlang der Längsachse verschoben werden und das Ventil des Vorratsbehälters damit betätigen und öffnen. Über das Betätigungselement gelangt das über das Ventil aus dem Vorratsbehälter austretende Behandlungsmittel zu einem von mehreren Anschlusselementen, welches mit einem Hohlkörper gekoppelt ist. Um einem Hohlkörper Behandlungsmittel zuzuführen, muss der Hohlkörper mit einem der Anschlusselemente des Adapters gekoppelt werden, der Hohlkörper und der Vorratsbehälter fixiert und gleichzeitig das Betätigungselement des Adapters in Richtung Vorratsbehälter gedrückt werden.

Die WO 97/48424 A1 beschreibt eine Vorrichtung zur Beaufschlagung eines Hohlkörpers mit Behandlungsmittel mit einem elektronisch ansteuerbaren Ventilblock.

Demgegenüber ist es insbesondere die Aufgabe der Erfindung, einen Adapter zur Kopplung eines Hohlkörpers im medizinischen Bereich mit einem Vorratsbehälter für ein Behandlungsmittel vorzuschlagen, mittels welchem auf einfache Weise dem Hohlkörper Behandlungsmittel aus dem Vorratsbehälter zugeführt werden kann. Erfindungsgemäß wird die Aufgabe mit einem Adapter zur Kopplung eines Hohlkörpers im medizinischen Bereich mit einem Vorratsbehälter für ein Behandlungsmittel mit den Merkmalen des Anspruchs 1 gelöst.

Der erfindungsgemäße Adapter zur Kopplung eines Hohlkörpers im medizinischen Bereich mit einem Vorratsbehälter für ein Behandlungsmittel verfügt über ein Haltelement, ein Betätigungselement, ein mit dem Betätigungselement verbundenes Anschlusselement zum Anschließen des Hohlkörpers und eine Führungskappe mit einer Führungseinrichtung. Das Halteelement ist so ausgeführt, dass es am Vorratsbehälter unbeweglich angeordnet werden kann. Das Betätigungselement ist so ausgeführt und angeordnet, dass es ein Ventil des Vorratsbehälters betätigen und über das Ventil austretendes Behandlungsmittel an das Anschlusselement weiterleiten kann. Das Betätigungselement kann eine Passiv-Stellung und eine Aktiv-Stellung einnehmen, wobei das Ventil des Vorratsbehälters in der Passiv-Stellung geschlossen und in der Aktiv-Stellung geöffnet ist. Erfindungsgemäß ragt das Anschlusselement durch die Führungskappe hindurch und die Führungskappe ist so gegenüber dem Halteelement, dem Betätigungselement und dem Anschlusselement angeordnet, dass durch ein von der Führungseinrichtung geführtes Verkippen des Anschlusselements gegenüber dem Haltelement, also gegenüber der Längsachse des Vorratsbehälters, das Betätigungselement von seiner Passiv-Stellung in seine Aktivstellung und umgekehrt bringbar ist.

Um dem Hohlkörper Behandlungsmittel zuzuführen, es als beispielsweise nach einer Reinigung mit Pflege-Öl zu schmieren, muss man lediglich den Vorratsbehälter mit dem an ihm angeordneten erfindungsgemäßen Adapter in eine Hand nehmen, den Hohlkörper mit der anderen Hand auf das Anschlusselement des Adapters aufstecken und damit anschließen und den Hohlkörper gegenüber der Längsachse des Vorratsbehälters kurz verkippen. Durch das Verkippen wird das Ventil des Vorratsbehälters betätigt und Behandlungsmittel strömt vom Vorratsbehälter über den Adapter zum Hohlkörper. Es muss nicht zusätzlich mit einem Finger auf einen Knopf gedrückt oder mit einer anderen Aktion das Ventil des Vorratsbehälters betätigt werden. Das durch die Führungseinrichtung geführte Verkippen allein genügt, um das Ventil des Vorratsbehälters zu betätigen und dem Hohlkörper Behandlungsmittel zuzuführen. Die Handhabung eines mit einem erfindungsgemäßen Adapter versehenen Vorratsbehälters ist damit besonders einfach und ergonomisch.

Das Haltelement ist insbesondere so ausgeführt, dass es auf einen im oberen Bereich des Vorratsbehälters umlaufenden Bund aufgesteckt oder aufgeklipst werden kann. Im Folgenden wird in Richtung Vorratsbehälter als unten und die entgegengesetzte Richtung als oben bezüglich des Halteelements und damit des Adapters bezeichnet. Das Haltelement weist insbesondere eine hauptsächlich zylinderförmige Grundform auf, welche im unteren Bereich an den Querschnitt des Vorratsbehälters und den genannten umlaufenden Bund angepasst ist.

Das Haltelement ist in seinem oberen Bereich mit dem Betätigungselement verbunden. Das Betätigungselement verfügt über ein hauptsächlich hohlzylindrisches, in einer Axialrichtung ausgerichtetes Durchgangselement und ein Verbindungsteil. Das Verbindungsteil stellt dabei die Verbindung mit dem Haltelement her, wobei die genannte Verbindung insbesondere über einen schmalen Verbindungssteg zwischen Halteelement und Betätigungselement ausgeführt ist. Das Durchgangselement erstreckt sich nach unten in Richtung Vorratsbehälter. Nach oben, also vom Vorratsbehälter weg, ist das Durchgangselement koaxial mit dem hauptsächlich hohlzylinderförmigen Anschlusselement verbunden. Das Anschlusselement ist damit ebenfalls in eine Axialrichtung ausgerichtet. Das Anschlusselement und das Betätigungselement sind dabei insbesondere einstückig, beispielsweise als ein Kunststoffspritzteil ausgeführt.

Insbesondere sind das Halteelement, das Betätigungselement und das Anschlusselement einstückig ausgeführt. Damit ist der Adapter besonders kostengünstig herstellbar. Das Halteelement, das Betätigungselement und das Anschlusselement sind dabei insbesondere als ein Kunststoffspritzteil ausgeführt.

Das Durchgangselement des Betätigungselements ist so angeordnet, dass es oben auf dem oben beschriebenen Röhrchen des Ventils des Vorratsbehälters aufsitzt und damit das Röhrchen des Ventils in Richtung Vorratsbehälter drücken und so das Ventil betätigen kann. In der Passiv-Stellung des Betätigungselements kann auch ein kleiner Abstand zwischen dem Durchgangselement und dem Röhrchen des Ventils vorhanden sein.

In der Passiv-Stellung des Betätigungselements drückt das Durchgangselement das Röhrchen des Ventils nicht in Richtung Vorratsbehälter, so dass der Vorratsbehälter verschlossen ist und kein Pflegemittel austreten kann. Das Durchgangselement des Betätigungselement ist in der Passiv-Stellung des Betätigungselements in Längsrichtung des Vorratsbehälters ausgerichtet. Die Axialrichtung des Durchgangselements und damit auch des Anschlusselements verläuft damit in Längsrichtung des Vorratsbehälters. Das Betätigungselement nimmt die Passivstellung dann ein, wenn keine Kraft auf das Betätigungselement, insbesondere über das Anschlusselement auf dessen Durchgangselement ausgeübt wird.

Das Halteelement wird von der Führungskappe so umschlossen, dass das Anschlusselement durch die Führungskappe nach oben hindurchragt. Die Führungskappe ist dazu fest mit dem Haltelement und/oder dem Vorratsbehälter verbunden. Die Führungskappe ist insbesondere auf einen umlaufenden Bund des Vorratsbehälters aufgesteckt oder aufgeklipst.

Damit das Anschlusselement durch die Führungskappe ragen kann, weist die Führungskappe eine insbesondere als eine Ausnehmung ausgeführte Führungseinrichtung auf. Die Führungseinrichtung ist insbesondere so ausgeführt, dass sie ausgehend von der Position des Anschlusselements in der Passiv-Stellung des Betätigungselements eine Bewegung des Anschlusselements gegenüber der Führungskappe nur in einer als Betätigungsrichtung bezeichneten Richtung zulässt. Die Führungskappe ist insbesondere ebenfalls als ein Kunststoffspritzteil ausgeführt, wobei die Führungskappe insbesondere nicht einstückig mit dem Haltelement, dem Betätigungselement und/oder dem Anschlusselement ausgeführt ist.

Um das Betätigungselement von seiner Passiv-Stellung in seine Aktiv-Stellung zu bringen, wird es gegenüber dem Halteelement in Betätigungsrichtung verkippt. Dazu wird insbesondere auf das Anschlusselement eine Kraft in der genannten Betätigungsrichtung und damit quer zur Längsrichtung des Vorratsbehälters ausgeübt. Damit wird über die starre Verbindung von Anschlusselement und Betätigungselement ein Drehmoment in das Betätigungselement eingeleitet. Die Verbindung zwischen Haltelement und dem Verbindungsteil des Betätigungselements ist so ausgeführt und angeordnet, dass das genannte Drehmoment zu einem Verkippen des Betätigungselement gegenüber dem Haltelement führt. Die Kippachse verläuft dabei quer zur Längsrichtung durch die genannte Verbindung zwischen Haltelement und Verbindungsteil des Betätigungselements, also beispielsweise durch den genannten Verbindungssteg zwischen Halteelement und Verbindungsteil des Betätigungselement. Die Kippachse verläuft also nicht durch das Durchgangselement des Betätigungselements, sondern neben dem Durchgangselement vorbei. Die Verbindung insbesondere in Form des Verbindungsstegs wird dabei elastisch verformt.

Das genannte Verkippen des Betätigungselements gegenüber dem Halteelement führt nicht nur dazu, dass sich das Durchgangselement des Betätigungselements gegenüber der Längsrichtung neigt, sondern auch dazu, dass ein unterer Endbereichs des Durchgangselements nach unten, also in Richtung Vorratsbehälter verlagert wird. Damit drückt das Durchgangselement des Betätigungselements beim genannten Verkippen das Röhrchen des Ventils nach unten und betätigt es damit. Beim Betätigen des Ventils wird die Druckfeder im Ventil zusammengedrückt. Das Röhrchen des Ventils des Vorratsbehälters verbiegt sich insbesondere nicht, d.h. es ist immer in Längsrichtung des Vorratsbehälters ausgerichtet. Damit das Durchgangselement das Ventil beim Verkippen des Betätigungselements gegenüber dem Halteelements dennoch sicher betätigen kann, ist insbesondere das Durchgangselement in seinem unteren, also in Richtung Vorratsbehälter orientieren Endbereich aufgeweitet. Der untere Endbereich kann beispielsweise einen konusförmigen oder einen halbkugelförmigen Verlauf aufweisen. Damit kann das Durchgangselement das Röhrchen des Ventils auch dann in Richtung Vorratsbehälter drücken, wenn es nicht mehr koaxial zum Röhrchen des Ventils ausgerichtet, sondern gegenüber dem Röhrchen geneigt angeordnet ist. Es ist aber auch möglich, dass der genannte Endbereich nicht aufgeweitet ist.

Sobald kein Drehmoment mehr in das Betätigungselement eingeleitet wird, bewegt sich das Betätigungselement auf Grund der genannten elastischen Verformung der Verbindung zwischen Haltelement und Verbindungsteil des Betätigungselements wieder in die Passiv-Stellung zurück. Es wirkt damit ein Rückstellmoment in Richtung seiner Passiv-Stellung auf das Betätigungselement. Das Durchgangselement des Betätigungselements richtet sich wieder in Längsrichtung aus und sein unterer Endbereich wird wieder nach oben, also vom Vorratsbehälter weg verlagert. Dadurch wird das Röhrchen des Ventils des Vorratsbehälters durch die Kraft der Druckfeder im Ventil nach oben gedrückt und damit geschlossen.

In Ausgestaltung der Erfindung ist die Führungseinrichtung der Führungskappe als eine längliche Ausnehmung in der Führungskappe ausgeführt, durch welche das Anschlusselement durch die Führungskappe hindurchragt. Das Anschlusselement wird beim genannten Verkippen des Anschlusselements gegenüber dem Haltelement an den seitlichen Rändern der Ausnehmung geführt. Die Führungseinrichtung ist damit besonders einfach und kostengünstig realisierbar.

Die genannte Ausnehmung der Führungskappe kann in Richtung Vorratsbehälter ragende Wangen aufweisen, welche die seitlichen Ränder der Ausnehmung ausbilden.

In Ausgestaltung der Erfindung ist die Führungseinrichtung so ausgeführt und angeordnet, dass sie das genannte Verkippen des Anschlusselements gegenüber dem Haltelement begrenzt. Damit kann vorteilhafterweise ein zu starkes Verkippen oder ein Verkippen in die falsche Richtung verhindert werden, was beides zu einer Beschädigung des Adapters führen könnte.

Die Führungseinrichtung ist dabei insbesondere so ausgeführt, dass sie ausgehend von der Passiv-Stellung des Betätigungselements nur ein Verkippen des Anschlusselements in die genannte Betätigungsrichtung zulässt. Außerdem begrenzt die Führungseinrichtung insbesondere auch das Verkippen des Anschlusselements in der Betätigungsrichtung. Die Führungseinrichtung weist also insbesondere zwei Anschläge auf, einen ersten Anschlag entgegen der Betätigungsrichtung und einen zweiten Anschlag in Betätigungsrichtung.

In Ausgestaltung der Erfindung ist die Führungseinrichtung als eine längliche Ausnehmung in der Führungskappe ausgeführt, durch welche das Anschlusselement durch die Führungskappe hindurchragt. Die längliche Ausnehmung der Führungskappe kann in Richtung Vorratsbehälter ragende Wangen aufweisen. Das genannte Verkippen des Anschlusselements gegenüber dem Haltelement wird am Ende der Ausnehmung, insbesondere an beiden Enden begrenzt. Die Führungseinrichtung ist damit besonders einfach und kostengünstig realisierbar.

In Ausgestaltung der Erfindung ist eine die Führungseinrichtung aufweisende und vom Betätigungselement wegweisende Deckfläche der Führungskappe geneigt gegenüber dem Betätigungselement in seiner Passivstellung angeordnet. Die Neigung ist dabei insbesondere in Betätigungsrichtung ausgerichtet, womit vorteilhafterweise die Betätigungsrichtung angezeigt wird.

Unter einer Neigung gegenüber dem Betätigungselement wird dabei eine Neigung gegenüber dem Verbindungselement des Betätigungselements verstanden. Damit schließt die geneigt angeordnete Deckfläche einen Winkel ungleich 90° mit der Längsrichtung des Vorratsbehälters ein.

Es ist aber auch möglich, dass die Deckfläche nicht geneigt gegenüber dem Verbindungselement des Betätigungselements angeordnet ist. Die Deckfläche schließt dann einen Winkel von 90° mit der Längsrichtung des Vorratsbehälters ein.

In Ausgestaltung der Erfindung weist die Führungskappe an ihrem dem Vorratsbehälter abgewandten Ende zwei Seitenwangen auf, zwischen denen sich die die Führungseinrichtung aufweisende und vom Betätigungselement wegweisende Deckfläche der Führungskappe erstreckt. Das Anschlusselement ist dabei vollständig zwischen den Seitenwangen angeordnet. Das Anschlusselement wird damit von den Seitenwangen, insbesondere bei einem Transport eines Vorratsbehälters mit an ihm angeordneten Adapter, vor Beschädigungen geschützt.

Unter einer vollständigen Anordnung zwischen den Seitenwangen soll hier insbesondere verstanden werden, dass das Anschlusselement nicht nach oben über die beiden Seitenwangen hinausragt. Die Seitenwangen sind dabei insbesondere gleich ausgeführt. Das Anschlusselement kann nach oben bündig mit den Seitenwangen abschließen oder die Seitenwangen können das Anschlusselement auch nach oben überragen.

In Ausgestaltung der Erfindung weist das Betätigungselement Torsionsstäbe auf, welche so ausgeführt und angeordnet sind, dass sie ein Rückstellmoment in Richtung Passiv-Stellung des Betätigungselements auf das Betätigungselement aufbringen. Damit wird eine zuverlässige Rückstellung des Betätigungselements aus seiner Aktiv-Stellung in seine Passiv-Stellung gewährleistet und damit ein ungewolltes Austreten von Pflegemittel aus dem Vorratsbehälter verhindert.

Das Betätigungselement weist dazu beispielsweise ein auf den ersten umlaufenden Bund des Vorratsbehälters aufgeklipbares Halteelement auf, welches hauptsächlich eine Ringform aufweist. Im Zentrum des Haltelements ist dann das Anschlusselement angeordnet. Das Anschlusselement erfüllt in diesem Fall alleine die Funktion des Durchgangselements und des Anschlusselements. Das Anschlusselement ist dann an gegenüberliegenden Seiten mit zwei koaxial angeordneten Torsionsstäben mit dem Halteelement verbunden.

In Ausgestaltung der Erfindung ist zumindest teilweise innerhalb des Anschlusselements ein Dichtelement angeordnet. Das Dichtelement überragt das Anschlusselement insbesondere nach oben, also in die vom Vorratsbehälter abgewandte Richtung. Das Dichtelement ist insbesondere so ausgeführt und angeordnet, dass es eine sichere und dichte Verbindung des Anschlusselements in Richtung Ventil des Vorratsbehälters, also insbesondere zum Röhrchen des Vorratsbehälters und/oder in Richtung Hohlkörper, also insbesondere zu einem auf das Anschlusselement aufsteckbares Endstück herstellen kann. Das Dichtelement verhindert damit ein ungewolltes Austreten des Pflegemittels an der Verbindung zwischen dem Adapter und dem Vorratsbehälters und/oder bei der Verbindung zwischen dem Adapter und dem Endstück.

Das Vorsehen eines Dichtelements bei einem Adapter zur Kopplung eines Hohlkörpers im medizinischen Bereich mit einem Vorratsbehälter für ein Behandlungsmittel kann auch ohne die oben beschriebene Führung durch die Führungskappe vorteilhaft sein und damit als eine eigenständige Erfindung angesehen werden. Damit würde sich ergeben:
Ein Adapter zur Kopplung eines Hohlkörpers im medizinischen Bereich mit einem Vorratsbehälter für ein Behandlungsmittel mit
   - einem Haltelement,
   - einem Betätigungselement und
   - einem mit dem Betätigungselement verbundenen Anschlusselement zum Anschließen des Hohlkörpers,
wobei
   - das Halteelement so ausgeführt ist, dass es am Vorratsbehälter unbeweglich angeordnet werden kann,
   - das Betätigungselement so ausgeführt und angeordnet ist, dass es ein Ventil des Vorratsbehälters betätigen und über das Ventil austretendes Behandlungsmittel an das Anschlusselement weiterleiten kann und
      eine Passiv-Stellung und eine Aktiv-Stellung einnehmen kann, wobei das Ventil des Vorratsbehälters in der Passiv-Stellung geschlossen und in der Aktiv-Stellung geöffnet ist und
   - zumindest teilweise innerhalb des Anschlusselements ein Dichtelement angeordnet ist, welches so ausgeführt und angeordnet ist, dass es eine dichte Verbindung des Anschlusselements in Richtung Ventil des Vorratsbehälters und/oder in Richtung Hohlkörper herstellen kann.

In Ausgestaltung der Erfindung bestehen das Anschlusselement und das Dichtelement aus unterschiedlichem Material. Das Dichtelement besteht insbesondere aus einem weicheren Material als das Anschlusselement, wobei insbesondere beide aus Kunststoff ausgeführt sind. Damit kann sowohl für das Anschlusselement als auch für das Dichtelements ein auf den Einsatzzweck abgestimmtes Material verwendet werden. Das Anschlusselement muss beispielsweise ausreichend steif ausgeführt sein und das Dichtelement gute Dichtungseigenschaften aufweisen.

In Ausgestaltung der Erfindung sind das Anschlusselement und das Dichtelement mittels eines Zwei Komponenten-Spritzgussverfahrens hergestellt. Dies ermöglicht eine besonders kostengünstige Herstellung des Anschlusselements und des Dichtelements. Dazu wird zunächst das Anschlusselement, insbesondere zusammen mit dem Haltelement aus einem ersten Kunststoff gespritzt und anschließend wird das Dichtelement an bzw. in das dadurch entstandene Bauteil aus einem zweiten, insbesondere weicheren Kunststoff gespritzt.

Alternativ dazu können zur Herstellung des Betätigungselement das Haltelement und das Anschlusselement einerseits und das Dichtelement andererseits als separate Bauteile hergestellt, insbesondere aus verschiedenen Kunststoffen gespritzt werden. Anschließend können die beiden Bauteile zusammengefügt werden.

In Ausgestaltung der Erfindung ist am Anschlusselement ein austauschbares Endstück angeordnet, über welches der Hohlkörper am Anschlusselement anschließbar ist. Damit lassen sich vorteilhafterweise Hohlkörper mit unterschiedlichen Öffnungen am erfindungsgemäßen Adapter anschließen.

Das genannte Endstück kann dabei insbesondere nur auf das Anschlusselement aufgesteckt werden. Um einen guten Halt des Endstücks zu ermöglichen, kann das Anschlusselement eine leicht konische Außenkontur aufweisen. Das Endstück ist insbesondere innen hohl, wobei eine Seite des Endstücks an die Außenkontur des Anschlusselements und die gegenüberliegende Seite an die Öffnung des Hohlkörpers angepasst ist. Das Endstück ist dabei ebenfalls insbesondere als ein Kunststoffspritzteil ausgeführt.

In Ausgestaltung der Erfindung ist das Endstück auf das Anschlusselement aufsteckbar und das Anschlusselement weist einen Anschlag für die Begrenzung des Aufsteckens des Endstücks in Richtung Betätigungselement auf. Der Anschlag ist insbesondere so positioniert, dass das Endstück sicher und korrekt auf das Anschlusselement aufgesteckt ist, wenn es den genannten Anschlag berührt. Damit kann sichergestellt werden, dass beim Gebrauch des Adapters das Endstück sicher und korrekt auf das Anschlusselement aufgesteckt ist und es sich nicht beim Zuführen von Behandlungsmittel zum Hohlkörper vom Anschlusselement löst.

Da unterschiedliche Hohlkörper unterschiedliche Öffnungen zum Anschließen an den erfindungsgemäßen Adapter aufweisen können, ist insbesondere für jeden anzuschließenden Typ von Hohlkörper ein angepasstes Endstück vorgesehen. Dabei sind die verschiedenen Endstücke insbesondere unterschiedlich gekennzeichnet, so dass jedem Typ von Hohlkörper ein Endstück mit einer spezifischen Kennzeichnung zugeordnet ist. Die verschiedenen Endstücke können als Kennzeichnung beispielsweise jeweils eine unterschiedliche Farbe oder eine unterschiedliche Beschriftung aufweisen.

Damit ergibt sich ein Adaptersystem zur Kopplung eines Hohlkörpers im medizinischen Bereich mit einem Vorratsbehälter für ein Behandlungsmittel mit einem oben beschriebenen Adapter und mehreren unterschiedlichen am Anschlusselement des Adapters anordenbaren Endstücken, über welche verschieden ausgeführte Hohlkörper am Anschlusselement anschließbar sind.

Ein derartiges Adaptersystem kann insbesondere zusätzlich einen Vorratsbehälter mit einem Behandlungsmittel umfassen.

Weitere Ausgestaltungen der Erfindung gehen aus der Beschreibung und der Zeichnung hervor. Ausführungsbeispiele der Erfindung sind in der Zeichnung vereinfacht dargestellt und in der nachfolgenden Beschreibung näher erläutert. Dabei zeigen
- Fig. 1: einen Schnitt durch einen mittels eines Adapters mit einem Hohlkörper im medizinischen Bereich gekoppelten Vorratsbehälter mit Behandlungsmittel mit geschlossenem Ventil,
- Fig. 2: ein Haltelement, ein Betätigungselement und ein Anschlusselement des Adapters aus Fig. 1 in einer Draufsicht,
- Fig. 3: eine Führungskappe des Adapters aus Fig. 1 in einer Draufsicht,
- Fig. 4: eine Seitenansicht der Führungskappe aus Fig. 3,
- Fig. 5: einen Schnitt durch den mittels des Adapters mit dem Hohlkörper im medizinischen Bereich gekoppelten Vorratsbehälter mit geöffnetem Ventil,
- Fig. 6: ein zweites Ausführungsbeispiel eines Haltelements und eines Anschlusselements in einer Draufsicht und
- Fig. 7: einen Schnitt durch das Haltelement und das Anschlusselement aus Fig. 6 entlang der Linie A-A.

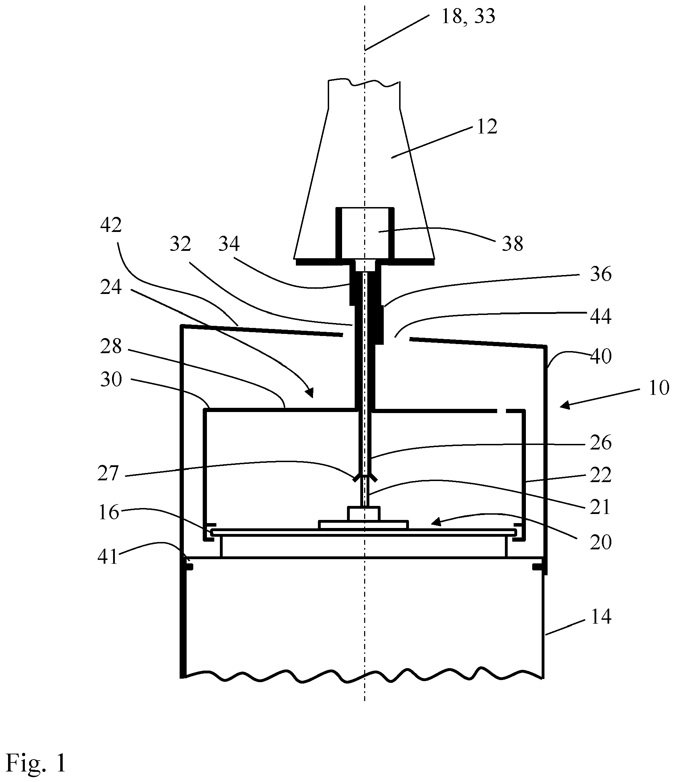

Gemäß Fig. 1 ist ein Adapter 10, mittels welchem ein Hohlkörper 12 im medizinischen Bereich mit einem Vorratsbehälter 14 für ein Behandlungsmittel gekoppelt, auf einen im oberen Bereich des Vorratsbehälters 14 ersten umlaufenden Bund 16 aufgeklipst. Der Hohlkörper 12 ist im vorliegenden Beispiel als eine Turbine ausgeführt. Der Vorratsbehälter 14 ist insbesondere als eine hauptsächlich zylinderförmige, mit einer Mischung aus Treibmittel und Pflege-Öl unter Druck gefüllte Spraydose ausgeführt. Der Vorratsbehälter 14 ist in einer Längsrichtung 18, welche seiner Axialrichtung entspricht, ausgerichtet. Er verfügt über ein Ventil 20 mit einem mittig angeordneten und in Längsrichtung verlaufenden hohlen Röhrchen 21. Das Ventil 20 ist in Fig. 1 geschlossen, so dass kein Pflegemittel aus dem Vorratsbehälter 14 austreten kann. Das Ventil 20 ist über den ersten umlaufenden Bund 16 mit dem restlichen Teil des Vorratsbehälters 14 verbunden.

Der Adapter 10 verfügt über ein hauptsächlich hohlzylinderförmiges Halteelement 22, über welches die Verbindung zum Vorratsbehälter 14 hergestellt ist. Das Haltelement 22 ist in seinem unteren Bereich an den Querschnitt des Vorratsbehälters 14 und an den ersten umlaufenden Bund 16 angepasst.

Das Haltelement 22 ist in seinem oberen Bereich mit einem Betätigungselement 24 verbunden. Das Betätigungselement 24 verfügt über ein hauptsächlich hohlzylindrisches Durchgangselement 26 und ein Verbindungsteil 28. Das Verbindungsteil 28 stellt dabei die Verbindung mit dem Haltelement 22 her. Das Betätigungselement 24 ist in Fig. 1 in seiner Passiv-Stellung, in der das Ventil 20 des Vorratsbehälters 14 geschlossen ist.

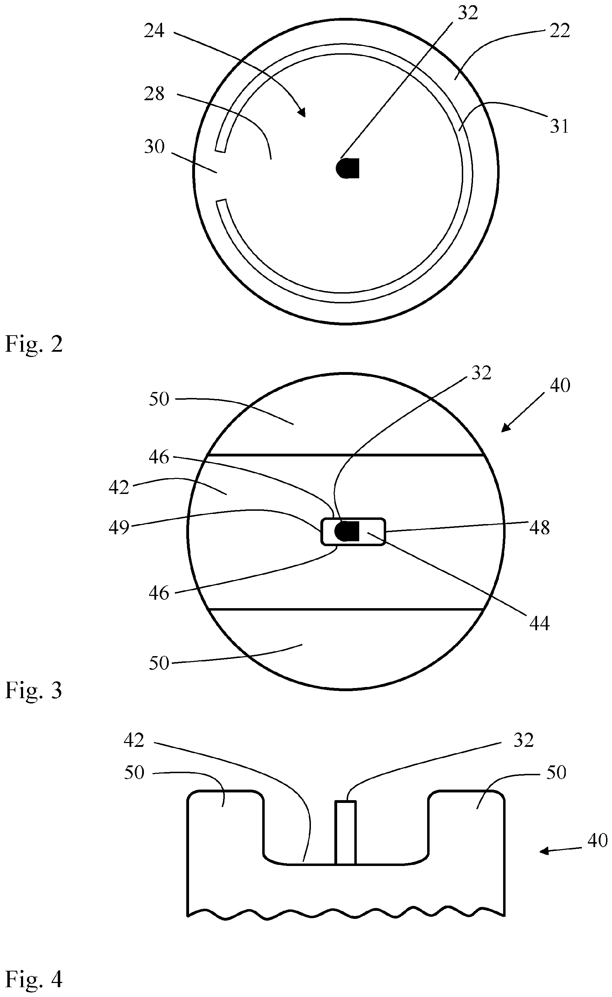

Die genannte Verbindung zwischen Haltelement 22 und Verbindungsteil 28 des Betätigungselements 24 ist in Fig. 2 in einer Draufsicht dargestellt. Gemäß Fig. 2 weist das Verbindungselement 28 des Betätigungselements 24 einen hauptsächlich kreisrunden Querschnitt auf und wird vollständig vom Haltelement 22 umschlossen. Die Verbindung zwischen Haltelement 22 und Verbindungselement 28 wird über einen schmalen Steg 30 hergestellt. Auf dem restlichen Umfang sind das Verbindungselement 28 und das Haltelement 22 durch einen entlang des Umfangs des Verbindungselements verlaufenden Spalt 31 voneinander getrennt.

Das Durchgangselement 26 des Betätigungselements 24 erstreckt sich gemäß Fig. 1 vom Verbindungselement 28 nach unten in Richtung Vorratsbehälter 14. Es ist in der in Fig. 1 dargestellten Passiv-Stellung des Betätigungselements 24 so koaxial zum Röhrchen 21 des Ventils 20 des Vorratsbehälters 14 angeordnet, dass es oben auf dem Röhrchen 21 des Ventils 20 aufsitzt. Eine Axialrichtung 33 des Verbindungselements 28 entspricht damit der Längsrichtung 18 des Vorratsbehälters 14. Es kann auch ein kleiner Abstand zwischen einem unteren Endbereich 27 des Durchgangselements 26 und dem Röhrchen 21 des Ventils 20 vorhanden sein. Der untere Endbereich 27 weitet sich nach unten leicht auf.

Nach oben, also vom Vorratsbehälter 14 weg, ist das Durchgangselement 26 koaxial mit einem hauptsächlich hohlzylinderförmigen Anschlusselement 32 verbunden. Das Anschlusselement 32 ist damit in der in Fig. 1 dargestellten Passiv-Stellung des Betätigungselements 24 ebenfalls in Axialrichtung 33 ausgerichtet. Das Anschlusselement 32, das Betätigungselement 24 und das Haltelement 22 sind dabei insbesondere einstückig, beispielsweise als ein Kunststoffspritzteil ausgeführt.

Am dem Vorratsbehälter 14 abgewandten Ende des Anschlusselements 32 ist ein hohlkörperförmiges Endstück 34 aufgesteckt. Es ist dabei so weit aufgesteckt, dass es einen nach außen gerichteten Anschlag 36 des Anschlusselements 32 berührt. Der Hohlkörper 12 ist auf ein dem Vorratsbehälter 14 abgewandtes Ende des Endstücks 34 aufgesteckt und damit mittelbar an das Anschlusselement 32 angeschlossen. Das dem Vorratsbehälter 14 abgewandte Ende des Endstücks 34 ist dabei auf eine Innenkontur einer Druckluftöffnung 38 des Hohlkörpers 12 angepasst.

Da unterschiedliche Hohlkörper unterschiedlich ausgeführte Druckluftöffnungen aufweisen können, können verschiedene, an den entsprechenden Hohlkörper angepasste Endstücke auf das Anschlusselement aufgesteckt werden. Dabei sind die verschiedenen Endstücke mittels unterschiedlicher Farben oder Beschriftungen unterschiedlich gekennzeichnet, so dass jedem Typ von Hohlkörper ein Endstück mit einer spezifischen Kennzeichnung zugeordnet ist.

Das Halteelement 22 und das Betätigungselement 24 sind vollständig innerhalb einer Führungskappe 40 angeordnet. Die Führungskappe 40 weist eine hauptsächlich zylinderförmige Außenkontur auf und ist in ihrem unten Bereich auf einen zweiten umlaufenden Bund 41 des Vorratsbehälters 14 aufgeklipst. Der zweite umlaufende Bund 41 des Vorratsbehälters 14 ist dabei unterhalb des ersten umlaufenden Bunds 16 angeordnet und weist einen größeren Durchmesser als dieser auf. An ihrem dem Vorratsbehälter 14 abgewandten Ende wird die Führungskappe 40 in dem in Fig. 1 dargestellten Schnitt von einer Deckfläche 42 abgeschlossen. Die vom Betätigungselement 24 wegweisende Deckfläche 42 ist in der Fig. 1 gegenüber dem Verbindungselement 28 des Betätigungselements 24 geneigt angeordnet. Die Neigung verläuft in der Fig. 1 von links nach rechts, was auch einer Betätigungsrichtung entspricht. Die Deckfläche 42 weist eine als eine Ausnehmung 44 ausgeführte Führungseinrichtung auf, durch welche das Anschlusselement 32 nach oben hindurchragt.

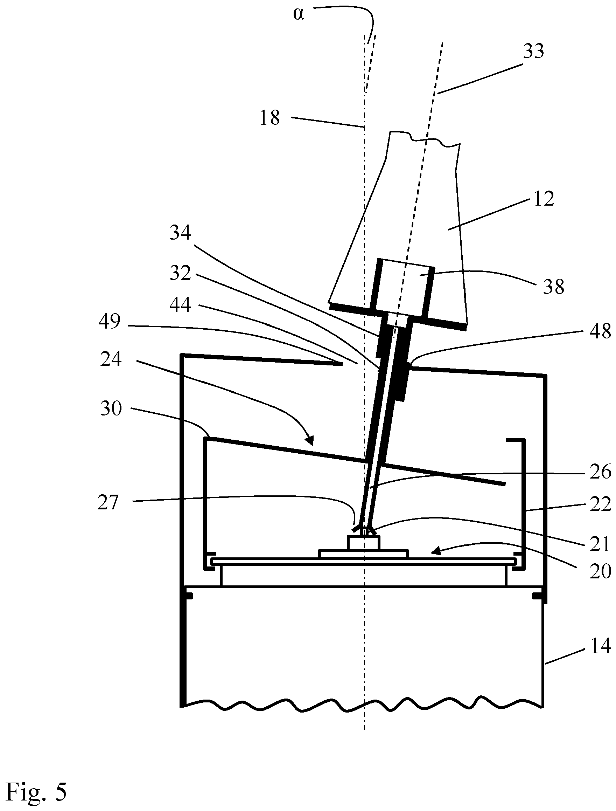

In Fig. 3 ist die Führungskappe 40 mit der Ausnehmung 44 und dem durch die Ausnehmung 44 hindurchragenden Anschlusselement 32 in einer Draufsicht dargestellt. Die Ausnehmung 44 weist einen hauptsächlich rechteckigen Querschnitt auf. Bei einem in Zusammenhang mit Fig. 5 beschriebenen Verkippen des Anschlusselements 32 gegenüber dem Haltelement 22 wird das Anschlusselement 32 von seitlichen Rändern 46 der Ausnehmung 44 geführt. Beim genannten Verkippen wird das Anschlusselement 32 in der Fig. 3 nach rechts und damit in Betätigungsrichtung verlagert. Die Verlagerung wird dabei von einem rechten Ende 48 der Ausnehmung begrenzt, womit das Ende 48 als ein Anschlag des Führungselements 44 bezeichnet werden kann. Die seitlichen Ränder 46 der Ausnehmung 44 sind dabei länger als das rechte Ende 48 und ein gegenüberliegendes linkes Ende 49, so dass die Ausnehmung als eine längliche Ausnehmung bezeichnet werden kann. Das linke Ende 49 der Ausnehmung 44 ist dabei so angeordnet, dass ein Verkippen des Anschlusselements 32 entgegen der Betätigungsrichtung, also in Fig. 3 nach links nicht möglich ist.

Die Führungskappe 40 weist zwei Seitenwangen 50 auf, welche parallel zu den seitlichen Rändern 46 der Ausnehmung 44 verlaufen. Zwischen den beiden Seitenwangen 50 ist die Deckfläche 42 angeordnet, welche die Ausnehmung 44 aufweist. Die Seitenwangen 50 der Führungskappe 40 sind in Fig. 4 in einer Seitenansicht dargestellt. Die Darstellung entspricht dabei einer Sicht auf den oberen Teil der Führungskappe 40 von links in Fig. 3. Die Seitenwangen 50 sind in zwei gegenüberliegenden äußeren Bereichen der Führungskappe 40 angeordnet und erstrecken sich weiter nach oben, also vom Vorratsbehälter 14 weg, als die zwischen ihnen angeordnete Deckfläche 42. Sie erstrecken sich so weit nach oben, dass das Anschlusselement 32 vollständig zwischen ihnen angeordnet ist. Die Seitenwangen 50 überragen dabei das Anschlusselement 32 ein wenig nach oben. Sie könnten aber auch bündig mit dem Anschlusselement abschließen.

Die Betätigung, also das Öffnen des Ventils 20 des Vorratsbehälters 14 wird im Folgenden in Zusammenhang mit Fig. 5 beschrieben. Das Ventil 20 wird geöffnet, also das Röhrchen 21 des Ventils 20 in Längsrichtung 18 gegen die Kraft einer nicht dargestellten Druckfeder in Richtung Vorratsbehälter 14 gedrückt, indem der Hohlkörper 12 und mit ihm das Endstück 34, das Anschlusselement 32 und das Betätigungselement 24 gegenüber dem Haltelement 22 und damit gegenüber der Längsrichtung 18 des Vorratsbehälters 14 in Betätigungsrichtung, also in Fig. 5 nach rechts verkippt wird. Das Verkippen führt dazu, dass die Axialrichtung 33 des Anschlusselements 32 nicht mehr in Längsrichtung 18 des Vorratsbehälters 14 verläuft, sondern einen Winkel α mit der Längsrichtung 18 des Vorratsbehälters 14 einschließt. Der Winkel α kann beispielsweise zwischen 2 und 15 °, insbesondere zwischen 3 und 8 ° betragen.

Beim Verkippen wird das Anschlusselement 32 von den seitlichen Rändern 46 der Führungseinrichtung in Form der Ausnehmung 44 geführt. Das Verkippen wird vom rechten Ende 48 der Ausnehmung 44 begrenzt. Das linke Ende 49 der Ausnehmung 44 verhindert ein Verkippen des Anschlusselements 32 nach links, also entgegen der Betätigungsrichtung. Durch das Verkippen wird der untere Endbereich 27 des Durchgangselements 26 des Betätigungselements 24 nach unten in Richtung Vorratsbehälter 14 verlagert und drückt damit das Röhrchen 21 des Ventils 20 in Längsrichtung 18 nach unten in Richtung Vorratsbehälter 14. Das Ventil 20 wird damit geöffnet, so dass Pflegemittel über das Röhrchen 21 des Ventils 20 aus dem Vorratsbehälter 14 austritt. Das Pflegemittel wird über das Durchgangselement 26, das Anschlusselement 32 und das Endstück 34 zur Druckluftöffnung 38 des Hohlkörpers 12 und damit in den Hohlkörper 12 geleitet. Das Betätigungselement 24 ist damit in Fig. 5 in seiner Aktiv-Stellung, in der das Ventil 20 des Vorratsbehälters 14 geöffnet ist.

Um das Austreten des Pflegemittels aus dem Vorratsbehälter 14 zu beenden, wird der Hohlkörper 12 und mit ihm das Endstück 34, das Anschlusselement 32 und das Betätigungselement 24 wieder in die in Fig. 1 dargestellte Position gebracht, so dass die Axialrichtung 33 des Anschlusselements 32 wieder in Längsrichtung 18 des Vorratsbehälters 14 verläuft. Das Betätigungselement 24 wird damit wieder von seiner Aktiv-Stellung in seine Passiv-Stellung gebracht.

Da durch die oben genannte Druckfeder, die beim Betätigen des Ventils 20 zusammengedrückt wird, eine Kraft nach oben auf das Röhrchen 21 des Ventils 20 wirkt und außerdem bei dem beschriebenen Verkippen des Anschlusselements 32 der schmale Steg 30 (siehe Fig. 2) als Verbindung zwischen Haltelement 22 und Verbindungselement 28 des Betätigungselements 24 elastisch verformt wurde, muss zur Einstellung der Passiv-Stellung des Betätigungselements 24 und damit zum Schließen des Ventils 20 des Vorratsbehälters 14 keine Kraft aufgebracht werden, sondern nur die Einleitung der zum Verkippen notwendigen Kraft beendet werden. Die elastische Verformung des Stegs 30 führt damit zu einem Rückstellmoment in Richtung Passiv-Stellung des Betätigungselements 24 auf das Betätigungselement 24.

Wie oben beschrieben, können auf das Anschlusselement des Adapters unterschiedliche Endstücke für unterschiedliche Hohlkörper aufgesteckt werden. Ein Adapter mit mehreren unterschiedlichen Endstücken bildet ein Adaptersystem zur Kopplung eines Hohlkörpers im medizinischen Bereich mit einem Vorratsbehälter für ein Behandlungsmittel. Das Adaptersystem kann außerdem den Vorratsbehälter mit Behandlungsmittel umfassen.

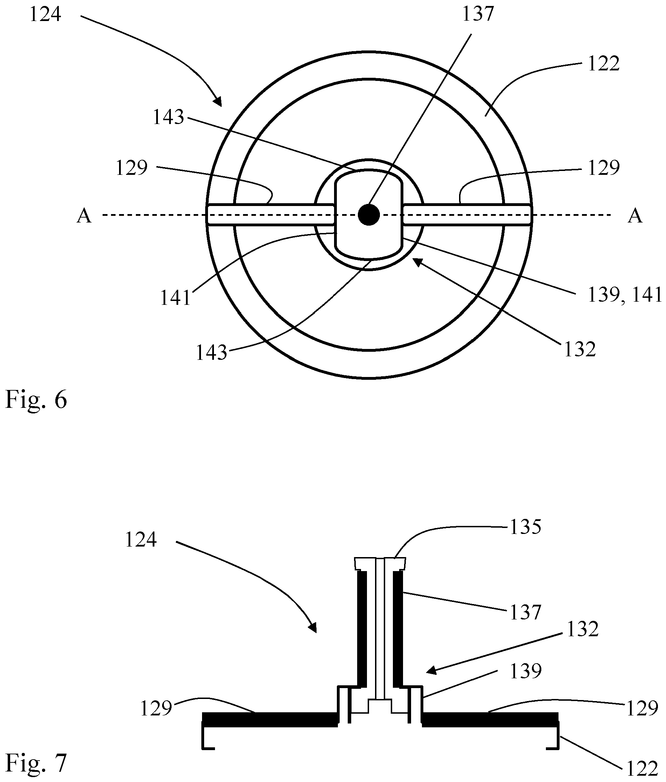

Bei einem zweiten Ausführungsbeispiels eines Betätigungselements 124 gemäß Fig.6 und 7 weist das Halteelement 122, welches auf den ersten umlaufenden Bund des Vorratsbehälters aufgeklipst werden kann, hauptsächlich eine Ringform auf, in dessen Zentrum das Anschlusselement 132 angeordnet ist. Das Anschlusselement 132 erfüllt dabei alleine die Funktion des Durchgangselements 26 und des Anschlusselements 32 des ersten Ausführungsbeispiels gemäß den Fig. 1 bis 5. Das Anschlusselement 132 ist an gegenüberliegenden Seiten mit zwei koaxial angeordneten Torsionsstäben 129 mit dem Halteelement 122 verbunden. Die beiden Torsionsstäbe 129 dienen dabei zum Aufbringen des oben genannten Rückstellmoments in Richtung Passiv-Stellung des Betätigungselements 124.

Bei einem Adapter mit einem Betätigungselement 124 gemäß Fig.6 und 7 erfolgt die Führung durch die Führungskappe ebenfalls gegenüber dem Anschlusselement 132 und auch der Anschlag der Führungskappe wirkt ebenfalls mit dem Anschlusselement 132 zusammen. Das Anschlusselement 132 weist dazu einen oberen, hauptsächlich hohlzylinderförmig ausgeführten Teil 137 und einen unteren Teil 139 zur Führung durch die Führungskappe auf. Der untere Teil 139 weist mit zwei ebene, sich gegenüberliegende parallele Flächen 141 zur Führung durch die Führungskappe und zwei gebogenen, sich gegenüberliegende Flächen 143 auf. In der Aktiv-Stellung des Betätigungselements 124 liegt eine der gebogenen Flächen 143 an der Führungskappe an und in der Passiv-Stellung des Betätigungselements 124 die andere, gegenüberliegende Fläche 143.

Die Führungskappe kann zur Führung des Anschlusselements 132 insbesondere in Richtung Vorratsbehälter ragende Wangen aufweisen, welche die in Zusammenhang mit Fig. 2 genannten seitlichen Ränder zum Führen des Anschlusselements 132 und den Anschlag zum Begrenzen der Verlagerung des Anschlusselements 132 ausbilden.

Teilweise innerhalb des Anschlusselements 132 ist ein Dichtelement 135 angeordnet, welches das Anschlusselement 132 nach oben überragt. Das Dichtelement 135 ist innen hohl, so dass es das aus dem Vorratsbehälter über das Röhrchen des Ventils austretende Pflegemittel in Richtung hohlkörperförmiges Endstück leiten kann. Es besteht aus einem Kunststoff, der weicher ist als der Kunststoff, aus welchem das Haltelement 122 und das Anschlusselement 132 bestehen, und weist insbesondere eine gute Dichtwirkung auf. Über das untere Ende des Dichtelements 135 wird die Verbindung zum Röhrchen des Ventils des Vorratsbehälters hergestellt, so dass eine sichere Verbindung zu dem Röhrchen hergestellt wird. Über das obere Ende des Dichtelements 135 wird die Verbindung zum hohlkörperförmigen Endstück hergestellt, so dass eine sichere Verbindung zum hohlkörperförmigen Endstück hergestellt wird.

Zur Herstellung des Betätigungselement 124 können das Haltelement 122 und das Anschlusselement 132 einerseits und das Dichtelement 135 andererseits als separate Bauteile hergestellt, insbesondere aus verschiedenen Kunststoffen gespritzt werden. Anschließend können die beiden Bauteile zusammengefügt werden.

Alternativ kann zunächst das Haltelement 122 und das Anschlusselement 132 aus einem ersten Kunststoff gespritzt werden und anschließend das Dichtelement 135 an bzw. in das dadurch entstandene Bauteil aus einem zweiten, weicheren Kunststoff gespritzt werden. Das Betätigungselement kann damit mit einem so genannten Zwei Komponenten-Spritzgussverfahren hergestellt werden.

Abschließend ist darauf hinzuweisen, dass Begriffe wie "aufweisend", "umfassend", etc. keine anderen Elemente oder Schritte ausschließen und Begriffe wie "eine" oder "ein" keine Vielzahl ausschließen. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Adapter zur Kopplung eines Hohlkörpers im medizinischen Bereich mit einem Vorratsbehälter für ein Behandlungsmittel mit
- einem Haltelement (22, 122),
- einem Betätigungselement (24, 124),
- einem mit dem Betätigungselement (24, 124) verbundenen Anschlusselement (32, 132) zum Anschließen des Hohlkörpers (12) und
- einer Führungskappe (40) mit einer Führungseinrichtung (44),
wobei
- das Halteelement (22, 122) so ausgeführt ist, dass es am Vorratsbehälter (14) unbeweglich angeordnet werden kann,
- das Betätigungselement (24, 124) so ausgeführt und angeordnet ist, dass es ein Ventil (20) des Vorratsbehälters (14) betätigen und über das Ventil (20) austretendes Behandlungsmittel an das Anschlusselement (32, 132) weiterleiten kann und
eine Passiv-Stellung und eine Aktiv-Stellung einnehmen kann, wobei das Ventil (20) des Vorratsbehälters (14) in der Passiv-Stellung geschlossen und in der Aktiv-Stellung geöffnet ist,
**dadurch gekennzeichnet, dass**
- das Anschlusselement (32, 132) durch die Führungskappe (40) hindurchragt und
- die Führungskappe (40) so gegenüber dem Halteelement (22, 122), dem Betätigungselement (24, 124) und dem Anschlusselement (32, 132) angeordnet ist, dass durch ein von der Führungseinrichtung (44) geführtes Verkippen des Anschlusselements (32, 132) gegenüber dem Halteelement (22, 122) das Betätigungselement (24, 124) von seiner Passiv-Stellung in seine Aktivstellung und umgekehrt bringbar ist.

2. Adapter nach Anspruch 1,
**dadurch gekennzeichnet, dass**
die Führungseinrichtung (44) als eine längliche Ausnehmung (44) in der Führungskappe (40) ausgeführt ist, durch welche das Anschlusselement (32, 132) durch die Führungskappe (40) hindurchragt und
an deren seitlichen Rändern (46) das Anschlusselement (32, 132) beim genannten Verkippen des Anschlusselements (32, 132) gegenüber dem Haltelement (22, 122) geführt wird.

3. Adapter nach Anspruch 1 oder 2,
**dadurch gekennzeichnet, dass**
die Führungseinrichtung (44) so ausgeführt und angeordnet ist, dass sie das genannte Verkippen des Anschlusselements (32, 132) gegenüber dem Haltelement (22, 122) begrenzt.

4. Adapter nach Anspruch 3,
**dadurch gekennzeichnet, dass**
die Führungseinrichtung (44) als eine längliche Ausnehmung (44) in der Führungskappe (40) ausgeführt ist, durch welche das Anschlusselement (32, 132) durch die Führungskappe (40) hindurchragt und
an deren Enden (48, 49) das genannte Verkippen des Anschlusselements (32, 132) gegenüber dem Haltelement (22, 122) begrenzt wird.

5. Adapter nach einem der Ansprüche 1 bis 4,
**dadurch gekennzeichnet, dass**
eine die Führungseinrichtung (44) aufweisende und vom Betätigungselement (24, 124) wegweisende Deckfläche (42) der Führungskappe (40) geneigt gegenüber dem Betätigungselement (24, 124) in seiner Passivstellung angeordnet ist.

6. Adapter nach einem der Ansprüche 1 bis 5,
**dadurch gekennzeichnet, dass**
die Führungskappe (40) an ihrem dem Vorratsbehälter (14) abgewandten Ende zwei Seitenwangen (50) aufweist, zwischen denen sich die die Führungseinrichtung (44) aufweisende und vom Betätigungselement (24, 124) weg weisende Deckfläche (42) der Führungskappe (40) erstreckt und das Anschlusselement (32, 132) vollständig zwischen den Seitenwangen (50) angeordnet ist.

7. Adapter nach einem der Ansprüche 1 bis 6,
**dadurch gekennzeichnet, dass**
das Betätigungselement (124) Torsionsstäbe (129) aufweist, welche so ausgeführt und angeordnet sind, dass sie ein Rückstellmoment in Richtung Passiv-Stellung des Betätigungselements (124) auf das Betätigungselement (124) aufbringen.

8. Adapter nach einem der Ansprüche 1 bis 7,
**dadurch gekennzeichnet, dass**
zumindest teilweise innerhalb des Anschlusselements (132) ein Dichtelement (135) angeordnet ist.

9. Adapter nach Anspruch 8,
**dadurch gekennzeichnet, dass**
das Anschlusselement (132) und das Dichtelement (135) aus unterschiedlichem Material bestehen.

10. Adapter nach Anspruch 9,
**dadurch gekennzeichnet, dass**
das Anschlusselement (132) und das Dichtelement (135) mittels einem Zwei Komponenten-Spritzgussverfahren hergestellt sind.

11. Adapter nach einem der Ansprüche 1 bis 10,
**dadurch gekennzeichnet, dass**
am Anschlusselement (32, 132) ein austauschbares Endstück (34) angeordnet ist, über welches der Hohlkörper (12) am Anschlusselement (32, 132) anschließbar ist.

12. Adapter nach Anspruch 11,
**dadurch gekennzeichnet, dass**
das Endstück (34) auf das Anschlusselement (32, 132) aufsteckbar ist und das Anschlusselement (32) einen Anschlag (36) für die Begrenzung des Aufsteckens des Endstücks (34) in Richtung Betätigungselement (24) aufweist.

13. Adaptersystem zur Kopplung eines Hohlkörpers im medizinischen Bereich mit einem Vorratsbehälter für ein Behandlungsmittel mit
einem Adapter (10) nach einem der Ansprüche 1 bis 10 und
mehreren unterschiedlichen am Anschlusselement (32) des Adapters (10) anordenbaren Endstücken (34), über welche verschieden ausgeführte Hohlkörper (12) am Anschlusselement (32, 132) anschließbar sind.

14. Adaptersystem nach Anspruch 13,
wobei das Adaptersystem einem Vorratsbehälter (14) mit Behandlungsmittel aufweist.

## Claims

1. Adapter for coupling a hollow body in the medical field with a storage container for a treatment agent with
- a retaining element (22, 122),
- an actuating element (24, 124),
- a connecting element (32, 132) connected to the actuating element (24, 124) for connecting the hollow body (12) and
- a guide cap (40) with a guide device (44),
whereby
- the retaining element (22, 122) is designed in such a way that it can be arranged immovably on the storage container (14),
- the actuating element (24, 124) is designed and arranged in such a way that it can actuate a valve (20) of the storage container (14) and pass on treatment agent emerging via the valve (20) to the connecting element (32, 132), and
can assume a passive position and an active position, wherein the valve (20) of the storage container (14) is closed in the passive position and open in the active position,
**characterized in that**
- the connecting element (32, 132) protrudes through the guide cap (40) and
- the guide cap (40) is arranged relative to the retaining element (22, 122), the actuating element (24, 124) and the connecting element (32, 132) in such a way that the actuating element (24, 124) can be brought from its passive position into its active position and vice versa by tilting the connecting element (32, 132) relative to the retaining element (22, 122) as guided by the guide device (44).

2. Adapter according to claim 1,
**characterized in that**
the guide device (44) is designed as an elongate recess (44) in the guide cap (40), through which the connecting element (32, 132) projects through the guide cap (40) and
at the lateral edges (46) of which the connecting element (32, 132) is guided during the said tilting of the connecting element (32, 132) relative to the retaining element (22, 122).

3. Adapter according to claim 1 or 2,
**characterized in that**
the guide device (44) is designed and arranged in such a way that it limits said tilting of the connecting element (32, 132) relative to the retaining element (22, 122).

4. Adapter according to claim 3,
**characterized in that**
the guide device (44) is designed as an elongate recess (44) in the guide cap (40), through which the connecting element (32, 132) projects through the guide cap (40) and
at the ends (48, 49) of which the said tilting of the connecting element (32, 132) relative to the retaining element (22, 122) is limited.

5. Adapter according to one of claims 1 to 4,
**characterized in that**
a cover surface (42) of the guide cap (40), which has the guide device (44) and points away from the actuating element (24, 124), is arranged inclined with respect to the actuating element (24, 124) in its passive position.

6. Adapter according to one of claims 1 to 5,
**characterized in that**
the guide cap (40) has two side cheeks (50) at its end facing away from the storage container (14), between which the cover surface (42) of the guide cap (40), which has the guide device (44) and faces away from the actuating element (24, 124), extends and the connecting element (32, 132) is arranged completely between the side cheeks (50).

7. Adapter according to one of claims 1 to 6,
**characterized in that**
the actuating element (124) has torsion bars (129) which are designed and arranged such that they apply a restoring torque to the actuating element (124) in the direction of the passive position of the actuating element (124).

8. Adapter according to one of claims 1 to 7,
**characterized in that**
a sealing element (135) is arranged at least partially inside the connecting element (132).

9. Adapter according to claim 8,
**characterized in that**
the connecting element (132) and the sealing element (135) are made of different materials.

10. Adapter according to claim 9,
**characterized in that**
the connecting element (132) and the sealing element (135) are manufactured by means of a two-component injection molding process.

11. Adapter according to one of claims 1 to 10,
**characterized in that**
an exchangeable end piece (34) is arranged on the connecting element (32, 132), via which end piece the hollow body (12) can be connected to the connecting element (32, 132).

12. Adapter according to claim 11,
**characterized in that**
the end piece (34) can be plugged onto the connecting element (32, 132) and the connecting element (32) has a stop (36) for limiting the plugging-on of the end piece (34) in the direction of the actuating element (24).

13. Adapter system for coupling a hollow body in the medical field to a storage container for a treatment agent, comprising
an adapter (10) according to one of claims 1 to 10
a plurality of different end pieces (34) which can be arranged on the connecting element (32) of the adapter (10) and via which differently designed hollow bodies (12) can be connected to the connecting element (32, 132).

14. Adapter system according to claim 13,
wherein the adapter system comprises a storage container (14) with treatment agent.

## Revendications

1. Adaptateur pour le couplage d'un corps creux dans le domaine médical avec un réservoir pour un produit de traitement avec
- un élément de retenue (22, 122),
- un élément d'actionnement (24, 124),
- un élément de raccordement (32, 132) relié à l'élément d'actionnement (24, 124) pour raccorder le corps creux (12) et
- d'un capuchon de guidage (40) avec un dispositif de guidage (44),
où
- l'élément de retenue (22, 122) est réalisé de telle sorte qu'il peut être disposé de manière immobile sur le réservoir (14),
- l'élément d'actionnement (24, 124) est réalisé et disposé de telle sorte qu'il peut actionner une soupape (20) du réservoir (14) et transmettre à l'élément de raccordement (32, 132) le produit de traitement qui sort par la soupape (20), et
peut prendre une position passive et une position active, la soupape (20) du réservoir (14) étant fermée dans la position passive et ouverte dans la position active,
**caractérisé en ce que**
- l'élément de raccordement (32, 132) fait saillie à travers le capuchon de guidage (40) et
- le capuchon de guidage (40) est disposé par rapport à l'élément de retenue (22, 122), à l'élément d'actionnement (24, 124) et à l'élément de raccordement (32, 132) de telle sorte que, par un basculement de l'élément de raccordement (32, 132) guidé par le dispositif de guidage (44) par rapport à l'élément de retenue (22, 122), l'élément d'actionnement (24, 124) peut être amené de sa position passive dans sa position active et inversement.

2. Adaptateur selon la revendication 1,
**caractérisé en ce que**
le dispositif de guidage (44) est réalisé sous la forme d'un évidement allongé (44) dans le capuchon de guidage (40), à travers lequel l'élément de raccordement (32, 132) fait saillie à travers le capuchon de guidage (40) et
sur les bords latéraux (46) duquel l'élément de raccordement (32, 132) est guidé lors dudit basculement de l'élément de raccordement (32, 132) par rapport à l'élément de retenue (22, 122).

3. Adaptateur selon la revendication 1 ou 2,
**caractérisé en ce que**
le dispositif de guidage (44) est réalisé et disposé de manière à limiter ledit basculement de l'élément de raccordement (32, 132) par rapport à l'élément de retenue (22, 122).

4. Adaptateur selon la revendication 3,
**caractérisé en ce que**
le dispositif de guidage (44) est réalisé sous forme d'un évidement allongé (44) dans le capuchon de guidage (40), à travers lequel l'élément de raccordement (32, 132) fait saillie à travers le capuchon de guidage (40) et
aux extrémités (48, 49) duquel est limité ledit basculement de l'élément de raccordement (32, 132) par rapport à l'élément de retenue (22, 122).

5. Adaptateur selon l'une quelconque des revendications 1 à 4,
**caractérisé en ce que**
une surface de recouvrement (42) du capuchon de guidage (40), présentant le dispositif de guidage (44) et s'éloignant de l'élément d'actionnement (24, 124), est disposée de manière inclinée par rapport à l'élément d'actionnement (24, 124) dans sa position passive.

6. Adaptateur selon l'une quelconque des revendications 1 à 5,
**caractérisé en ce que**
le capuchon de guidage (40) présente, à son extrémité opposée au réservoir (14), deux joues latérales (50) entre lesquelles s'étend la surface de recouvrement (42) présentant le dispositif de guidage (44) et orientée à l'opposé de l'élément d'actionnement (24, 124) du capuchon de guidage (40) et l'élément de raccordement (32, 132) est disposé entièrement entre les joues latérales (50).

7. Adaptateur selon l'une quelconque des revendications 1 à 6,
**caractérisé en ce que**
l'élément d'actionnement (124) comprend des barres de torsion (129) qui sont conçues et agencées de manière à appliquer un couple de rappel l'élément d'actionnement (124) en direction de la position passive de l'élément d'actionnement (124).

8. Adaptateur selon l'une quelconque des revendications 1 à 7,
**caractérisé en ce que**
un élément d'étanchéité (135) est disposé au moins partiellement à l'intérieur de l'élément de raccordement (132).

9. Adaptateur selon la revendication 8,
**caractérisé en ce que**
l'élément de raccordement (132) et l'élément d'étanchéité (135) sont constitués de matériaux différents.

10. Adaptateur selon la revendication 9,
**caractérisé en ce que**
l'élément de raccordement (132) et l'élément d'étanchéité (135) sont fabriqués au moyen d'un procédé de moulage par injection à deux composants.

11. Adaptateur selon l'une quelconque des revendications 1 à 10,
**caractérisé en ce que**
une pièce d'extrémité interchangeable (34) est disposée sur l'élément de raccordement (32, 132), par laquelle le corps creux (12) peut être raccordé à l'élément de raccordement (32, 132).

12. Adaptateur selon la revendication 11,
**caractérisé en ce que**
le pièce d'extrémité (34) peut être emboîté sur l'élément de raccordement (32, 132) et l'élément de raccordement (32) présente une butée (36) pour limiter l'emboîtement de l'embout (34) en direction de l'élément d'actionnement (24).

13. Système d'adaptateur pour le couplage d'un corps creux dans le domaine médical avec un réservoir pour un produit de traitement avec
un adaptateur (10) selon l'une des revendications 1 à 10
plusieurs pièces d'extrémité (34) différentes pouvant être disposées sur l'élément de raccordement (32) de l'adaptateur (10), par lesquelles des corps creux (12) de conception différente peuvent être raccordés à l'élément de raccordement (32, 132).

14. Système d'adaptation selon la revendication 13,
dans lequel le système d'adaptation comprend un réservoir (14) contenant un agent de traitement.
